# EUROPÄISCHE PATENTSCHRIFT

(11) **EP 0 791 342 B1**
(45) Veröffentlichungstag und Bekanntmachung des Hinweises auf die Patenterteilung: **28.07.1999**
(21) Anmeldenummer: 97100111.0
(22) Anmeldetag: 07.01.1997
(51) Int. Cl.: A61F 2/36, A61B 17/74

(54) **Schenkelhalsendoprothese für ein künstliches Hüftgelenk**
Femoral neck endoprosthesis for an artificial hip joint
Endoprothèse du col du fémur pour une articulation de hanche artificielle

(30) Priorität: 16.01.1996 DE 19601340
(43) Veröffentlichungstag der Anmeldung: 27.08.1997
(73) Patentinhaber: ESKA Implants GmbH & Co., 23556 Lübeck (DE)
(72) Erfinder: Grundei, Hans, Dr., 23558 Lübeck (DE); Scholz, Jörg, 14193 Berlin (DE)
(74) Vertreter: Fuchs Mehler Weiss & Fritzsche

(56) Entgegenhaltungen:
- EP-A- 0 099 167
- EP-A- 0 230 006
- EP-A- 0 561 263
- WO-A-93/01769
- DE-A- 3 607 824

## Beschreibung

Die Erfindung betrifft eine Schenkelhalsendoprothese für ein künstliches Hüftgelenk.

Zur Behandlung eines distruierten, d. h. zerstörten Hüftgelenkskopf, woraus erhebliche Schmerzempfindungen und gravierende funktionelle Einschränkungen des Gelenks resultieren, haben sich im wesentlichen zwei Therapiemöglichkeiten während der letzten Jahrzehnte durchgesetzt:

Zum einen ist es möglich, den Patienten mit einer Orthese zu versorgen, d. h. also mit einem externen Stützapparat. Neben der Tatsache, daß diese Versorgungsmöglichkeit kosmetisch völlig unakzeptabel ist, ist sie auch versorgungstechnisch nur suboptimal.

Als weitere, weitverbreitetere Möglichkeit ist vorgesehen, den Patienten mit einer Endoprothese zu versorgen, namentlich mit einer Hüftstielendoprothese, bei welcher ein Stiel in den zuvor freigeräumten Knochenmarksraum des Femurs eingesetzt wird und dort zementlos oder unter Verwendung eines Zements fixiert wird. An der proximalen Seite weist eine derartige Endoprothese dann die Möglichkeit des Anschlusses einer künstlichen Gelenkkugel vor.

Gerade die letzte Möglichkeit ist stark in der Patentliteratur vertreten. An dieser Stelle sei nur beispielhaft auf die DE-U-94 12 408 hingewiesen

Unter den einen oder anderen der in der Patentliteratur vorgeschlagenen Hüftstielendoprothesen gibt es einige Ansätze, die recht vielversprechend sind im Hinblick auf die Langzeitstabilität im Patientenkörper. Früher oder später jedoch, beispielsweise nach 10 bis 15 Jahren, ist zu beobachten, daß die Endoprothese einem Verschleiß unterworfen ist, welcher zu der Notwendigkeit führt, in einem Revisionseingriff die implantierte Endoprothese zu entfernen und durch eine neue zu ersetzen. Dies ist freilich nicht ganz unproblematisch, da durch die Resektion und Ausräumung des Knochenmarkraumes ein nicht unerheblicher Anteil natürlichen Knochenmaterials bei der Erstimplantation entfernt worden ist. Das einmal entfernte Material fehlt dann unter Umständen bei einem Revisionseingriff. Dies kommt insbesondere dann zum Tragen, wenn die Patienten relativ jung sind, bei denen also von vorn herein davon auszugehen ist, daß sie im Laufe ihres Lebens mindestens einem Revisionseingriff unterworfen werden müssen.

Eine Endoprothese, die mit einer geringfügigeren Resektion natürlichen Knochermaterials auskommt als die bekannten Hüftstielendoprothesen ist bekannt geworden aus der DE-A-27 24 234 sowie aus der EP-A-0099167. Das darin beschriebene Prothesenteil wird im oberen Bereich eines Femurs, aber unterhalb des Trochanter minors implantiert, ohne daß ein Stiel in den Knochermarksraum reichen würde. Diese auch als Druckscheibenprothese bezeichnete Prothese greift im wesentlichen mit einer proximalen Druckplatte über die Kortikalis des resezierten Femurs im Bereich des entfernten Hüftgelenkkopfes. Sie wird mit einer Druckplatte, die lateral am Femur anliegt, verspannt, derart, daß alle mechanischen Kräfte zwischen der Endoprothese und dem Femur direkt in die Kortikalschicht des Femurs eingeleitet werden, wodurch eine als unzulässig empfundene mechanische Beanspruchung der Spongiosa vermieden werden soll. Lediglich eine geringe Menge von Knochenzement ist für die Arretierung der Druckscheibe im proximalen Bereich notwendig.

Der Philosophie dieser Prothese liegt - wie ausgeführt - die Annahme zugrunde, daß die Spongiosa des Femurs möglichst wenig beansprucht werden soll. Dies wird erkauft durch eine extreme Belastung der Kortikalis des Femurs, indem nämlich sämtliche Kräfte darauf abgelastet und darin eingeleitet werden. Dies entspricht nach heutiger Erkenntnis in keiner Weise den natürlichen Belastungsverhältnissen.

Eine ganz ähnlich wirkende Prothese zeigt im übrigen die WO 89/11837, bei der die Funktion der proximalen Druckplatte gemäß der vorerwähnten Druckschrift wahrgenommen wird durch den speziell ausgebildeten Hüftgelenkkopf, der innenseitig mit einer Ausnehmung versehen ist, an der sich innenseitig der resizierte Femurstumpf unter Druckerzeugung anlegt.

Zwei Prothesen, bei denen augenscheinlich die Hauptlast von der Spongiosa des Femurknochens aufgenommen werden soll, sind bekanntgeworden aus der FR 26 26 169 A1 und der FR 26 74 122 A1. Bei der erstgenannten Druckschrift der beiden ist vorgesehen, einen proximal einen Steckkonus aufweisenden Gewindebolzen in die Spongiosa einzuschrauben. Dies dürfte innerhalb kürzester Zeit zu schweren Instabilitätsproblemen führen. Bei der zweitgenannten Druckschrift der beiden wird eine einen Steckkonus tragende Platte mittels einer Reihe von Knochenschrauben befestigt, wobei die Knochenschrauben in die Spongiosa reichen. Auch hieraus ergeben sich ernsthafte Stabilitätsfragen, da die Spongiosa von Natur aus im Verhältnis zur Kortikalis des Femurknochens punktuell nur gering belastbar ist.

Vor diesem Hintergrund ist es daher die Aufgabe der vorliegenden Erfindung, eine Schenkelhalsendoprothese für ein künstliches Hüftgelenk anzugeben, die sich hinsichtlich der Krafteinleitung in den Femur bei nur relativ geringfügig notwendig werdenden Resektionen von natürlichen Knochenmaterial den natürlichen Verhältnissen erheblich besser anpaßt als die vorerwähnte Prothese.

Gelöst wird diese Aufgabe dadurch, daß die Schenkelhalsendoprothese aufweist:
- eine zementlos im oberen Bereich eines Femurs unterhalb des Trochanter minors implantierbare Hülse, mit deren proximalen Ende ein Adapter zur Aufnahme einer künstlichen Gelenkkugel verbunden ist, wobei die Hülsenaußenseite zumindest teilweise mit einer offenmaschigen dreidimensionalen Raumnetzstruktur belegt ist, sowie
- eine Zugplatte, an der eine Zugschraube angeschlagen ist, die durch eine Durchbohrung im distalen Ende der Hülse in deren Inneres gesetzt und mit einem dort vorgesehenen Gewinde verschraubt ist, wobei
- sie proximalseitig keine Druckplatte zum Einleiten von Druckkräften auf die Kortikalis des Femurs aufweist.

Im Gegensatz zu der oben beschriebenen Druckscheibenendoprothese werden die Belastungskräfte nicht in die Kortikalis des Femurs eingeleitet, sondern direkt in die Spongiosa im Inneren des Femurs, nachdem Knochentrapekel während der Einheilungsphase durch die offenmaschige dreidimensionale Raumnetzstruktur auf der Hülsenaußenseite gewachsen ist. Es wird also mit der erfindungsgemäßen Endoprothese genau der Weg beschrieben, welcher in der DE-27 24 234 als nachteilig angesehen worden ist, nämlich die Spongiosa zu belasten. Nach der Einheilphase verhält sich die Hülse der Endoprothese in Nachbildung der natürlichen Verhältnisse aufgrund ihrer quasi Aufhängung an vielen Knochentrapekeln dynamisch, d. h. sie kann abhängig von den jeweiligen Belastungen ausweichende Bewegungen durchaus nachvollziehen und Ausgleichsbewegungen vollführen. Diese Art der Fixation einer Endoprothese entspricht den neueren Erkenntnissen aus der Chirurgie, daß nämlich in einem Röhrenknochen die Hauptlasten nicht von der Kortikalis, sondern von der Spongiosa aufgenommen werden. Es findet mit der erfindungsgemäßen Endoprothese keine Druckbeaufschlagung der Kortikalis statt.

Zur Vorbereitung der Implantation wird zunächst der zerstörte Femurkopf durch eine subcapitale Resektion entfernt, und zwar im wesentlichen orthograd zur Schenkelhalsachse. Sodann wird mittels eines Fräsers, der eine im wesentlichen gleiche Außenkontur wie die zu implantierende Hülse aufweist, in den Schenkelhals getrieben und eine entsprechende Bohrung oder Aushöhlung in der Spongiosa des Femurs hergestellt. Sodann kann die Hülse in den ausgefrästen Raum im Schenkelhals gesetzt werden. Zur lateralen Festlegung der Hülse ist vorgesehen, von außen an den Femur die erwähnte Zugplatte anzulegen, welche mittels einer Zugschraube mit dem Inneren der Hülse verschraubbar ist. Zu diesem Zweck muß die Kortikalis lateral aufgebohrt werden, so daß die Zugschraube durch die Kortikalis in das Innere der Hülse geführt werden kann, um dort verschraubt zu werden. Die Zugplatte bildet mit der Zugschraube ein im Prinzip bekanntes System der Zuggurtung.

Der mit dem proximalen Ende der Hülse zu verbindende Adapter kann beliebige geeignete Formen annehmen. Generell ist jedoch darauf zu achten, daß keinerlei Effekte wie bei der Druckscheibe entstehen, wie sie aus der genannten DE-A-27 24 234 bekannt sind.

Die Hülse sollte vorteilhafterweise im Querschnitt eine ovalförmige Gestalt haben. Hierdurch wird von vornherein eine Rotationsstabilität der Hülse in der Spongiosa erzielt.

Bevorzugt ist im übrigen die Ausbildung der Hülse derart, daß sie sich von ihrem proximalen Ende hin zu ihrem distalen Ende konisch verjüngt. Zum einen entspricht dies der mehr oder weniger konischen Ausbildung des Schenkelhalses, stellt also eine anatomische Adaption dar. Zum anderen wird durch Flächenpressung während der Einheilphase die Kräfteverteilung günstiger wie bei einer Osteosynthese, wenn die Hülse mit der Zugschraube verspannt ist und unter Belastung gesetzt wird, als wenn die Hülse beispielsweise zylindrisch ausgeformt ist.

Die Raumnetzstruktur auf der Hülsenaußenseite muß nicht von homogener Maschigkeit sein. Vielmehr wird gemäß einer vorteilhaften Weiterbildung vorgesehen, daß die Raumnetzstruktur auf den nach kaudal und nach kranial (also nach oben und nach unten) weisenden Hülsenaußenseiten grobmaschig mit Maschenweiten zwischen 3 bis 6 mm ausgebildet ist. Dies entspricht den Hauptbelastungsrichtungen der Hülse in dem natürlichen Spongiosabett im Femur und den Trajektorien des Spongiosagewebes des natürlichen Implantat-Lagers im Femur.

Als eine weitere anatomische Adaption ist vorteilhaft vorgesehen, daß die Raumnetzstruktur in Richtung ventral und dorsal auf der Hülsenaußenseite feinmaschig mit Maschenweiten zwischen 1 bis 2,5 mm ausgebildet ist. Die bereits angesprochene Ovalform des Querschnittes der Hülse ergibt sich bei der Ausbildung der Raumnetzstruktur auf der Hülsenaußenseite mit zwei unterschiedlichen Maschenweiten in diesem Falle von selbst. In diesem Falle wäre die größere Hauptachse des Oval zu suchen in den Bereichen, in denen die grobmaschige Raumnetzstruktur angeordnet ist, die kleinere Nebenachse hingegen bei den kleineren Maschenweiten.

Bei besonders bevorzugter Ausbildung ist vorgesehen, daß der Adapter für die Gelenkkugel im wesentlichen als Doppelsteckkonus ausgebildet ist mit einem an den beiden Konusbasen umlaufenden Flansch, wobei im proximalen Bereich der Hülse eine dem einen Steckkonus entsprechend ausgebildete konische Steckhülse vorgesehen ist. Zwischen dem einen Steckkonus und der Steckhülse in der Hülse kann also eine konische Klemmverbindung nach bekannten Prinzipien hergestellt werden, wohingegen der andere Steckkonus mit einer Steckhülse in einem künstlichen Gelenkkopf zusammenarbeitet.

Zu bemerken ist hierbei folgendes: der an den beiden Konusbasen umlaufende Flansch dient nicht zur Anlage auf der Resektionsfläche, wie dies beispielsweise bei der proximalen Druckscheibe gemäß der DE-A 27 24 234 der Fall ist. Idealerweise befindet sich zwischen der Resektionsfläche und der dieser zugewandten Seite des Flansches nach Herstellung der konischen Klemmverbindung zur implantierten Hülse ein Spalt von etwa 1 mm Breite. Dieser wird nach der Implantation im Laufe der Zeit von Knochentrapekeln durchwachsen werden, wenn der Flansch auf seine nach außen weisenden Flächen mit einer offenmaschigen dreidimensionalen Raumnetzstruktur belegt ist. Diese Arretierung dient lediglich der Festlegung des Adapters, nicht der Krafteinleitung von Belastungskräften in die Kortikalis des Femurs. Alternativ wäre auch eine Festlegung mittels Schrauben denkbar.

Im Falle eines notwendig werdenden Revisionseingriffes paßt in den angesprochenen Spalt das Sägeblatt einer Säge, mit welcher die Knochentrapekel zwischen Adapter, Flansch und Resektionsfläche einfach durchtrennt werden können, so daß der Adapter aus der Hülse mittels eines Abziehwerkzeuges einfach abgezogen werden kann.

Der Adapter kann durch eine weitere vorteilhafte Maßnahme weiter in seinem konischen Klemmsitz gesichert werden, dadurch, daß im der Hülse zuzuwendenden Steckkonus des Adapters eine Sackbohrung mit Innengewinde vorgesehen ist, mit welchem das Ende der mit einem entsprechenden Gewindeabschnitt versehenen Zugschraube zur Arretierung verschraubbar ist. In diesem Falle greift also die Zugschraube durch das Innere der Hülse weit über das Gewinde hinaus, mit welchem die Zugplatte mit der Hülse mittels der Zugschraube verschraubbar ist, nämlich bis weit hinein in den Bereich der proximalen Steckhülse. Der Steckkonus des Adapters wird in diesem Falle also regelrecht in die konische Klemmhülse der Hülse im Femur durch die Zugschraube hineingezogen und dort unverrückbar festgelegt.

Damit es nach Herstellung der Verbindung zwischen Hülse und Zugplatte außen an der Kortikalis des Femurs nicht zu deren Lockerung kommt beim Herstellen der Verbindung zwischen dem Adapter und der Hülse, weist der Gewindeabschnitt am Ende der Zugschraube vorzugsweise eine andere Steigung auf als das Gewinde im Inneren der Hülse. Hierdurch wird ein Selbstkontereffekt erzielt, der eben eine Lockerung der zuerst hergestellten Verbindung unterbindet.

Gemäß einer noch weiteren vorteilhaften Ausführungsform der Schenkelhalsendoprothese ist vorgesehen, daß die den Femur zuzuwendenden Flächen der Zugplatte ebenfalls mit einer offenmaschigen dreidimensionalen Raumnetzstruktur belegt sind. Hierdurch erfolgt eine weitere Festlegung des Implantates gegenüber Mikrobewegungen bei Belastungen. Theoretisch könnte zwar auf die Zugplatte nach einem vollständigen Einheilen der Hülse im Spongiosaraum des Femurs verzichtet werden, da diese Fixationsart zu einem äußerst innigen Verbund zwischen Implantat und der Knochenumgebung führt. Überlegungen hinsichtlich einer Langzeitfixation und hinsichtlich der Vermeidung eines unnötigen Risikos können es jedoch erwünscht erscheinen lassen, diese zusätzliche Sicherungsmaßnahme an der Zugplatte vorzusehen.

Eine weitere Sicherungsmaßnahme hinsichtlich der Lage der Zugplatte an der lateralen Kortikalis des Femurs ist vorteilhaft dann gegeben, wenn die Zugplatte mit wenigstens einem in Richtung des Femurs weisenden Widerlager in Form eines Zapfens versehen ist, welcher mit der Zugplatte im wesentlichen denselben Winkel einschließt wie die Hauptachse der Hülse mit der Zugplatte, wobei unter der Achse der Zugplatte deren Hauptachse zu verstehen ist. Das Widerlager wirkt rotationshemmend. Gleichzeitig können etwaige Biegemomente abgelastet werden.

Die Erfindung wird anhand eines Ausführungsbeispiels näher erläutert. Hierbei zeigt:
- Fig. 1: die Schnittansicht einer im Femur zu implantierenden Hülse der Endoprothese,
- Fig. 2: die Schnittansicht der Zugplatte der Endoprothese,
- Fig. 3: die Ansicht der Zugschraube,
- Fig. 4: die Ansicht in Richtung des Pfeils IV in Fig. 1 auf das proximale Ende der Hülse,
- Fig. 5: die Ansicht des Adapters zur Aufnahme einer künstlichen Gelenkkugel, teilweise im Schnitt,
- Fig. 6: die Komponenten der Endoprothese aus den Figuren 1 bis 5 in schematisch dargestellter Lagebeziehung entsprechend der Lagebeziehung bei der Implantation, und
- Fig. 7: die schematisch dargestellte Implantationslage der Endoprothese in dem Schenkelhals eines Femurs.

Nachfolgend sind dieselben Teile mit denselben Bezugszeichen versehen.

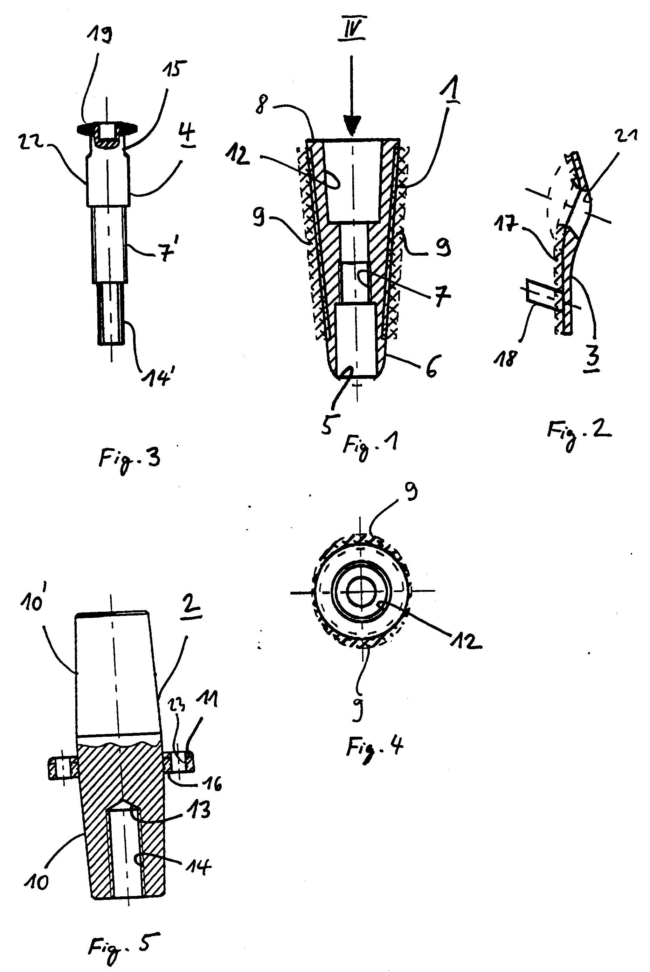

In Fig. 1 ist die Hülse 1 der erfindungsgemäßen Endoprothese dargestellt. Die Hülse weist ein proximales Ende 8 und ein distales Ende 6 auf. Im dargestellten Ausführungsbeispiel verläuft die Hülse von ihrem proximalen Ende 8 hin zu ihrem distalen Ende 6 sich konisch verjüngend, was die erwünschten Effekte hinsichtlich der Kraftverteilung bewirkt. Die im Schnitt dargestellte Hülse 1 ist auf ihren Außenflächen vorliegend mit einer offenmaschigen dreidimensionalen Raumnetzstruktur 9 relativ grober Maschenweite (3 bis 6 mm) belegt, und zwar auf den Hülsenaußenseiten, die nach Implantation im Femur nach kaudal und nach kranial zeigen. Dies entspricht in anatomischer Adaption in etwa den Größen der natürlichen Knochentrapekel in diesem im Schenkelhals.

Auf den nach ventral und dorsal weisenden Hülsenaußenseiten (Fig. 4) ist zwar ebenfalls eine offenmaschige dreidimensionale Raumnetzstruktur vorgesehen, jedoch mit wesentlich geringerer Maschenweite, wie dies in Fig. 4 schematisch dargestellt ist. Diese Maschenweiten betragen zwischen 1 bis 2,5 mm.

Im distalen Ende 6 der Hülse 1 ist eine Durchbohrung 5 vorgesehen, der sich in Richtung auf das proximale Ende 8 der Hülse 1 ein Innengewinde 7 anschließt. Dieses geht über in eine zylindrische Bohrung, welche schließlich in einer konischen Klemmhülse 12 am proximalen Ende 8 endet.

Eine weitere wichtige Komponente der Endoprothese ist die Zugplatte 3, wie sie in beispielhafter Ausführungsform in Fig. 2 in Schnittansicht gezeigt ist. Sie besteht im wesentlichen aus einem plattenförmigen Element, welches eine Durchbohrung 21 aufweist zur Durchführung einer Zugschraube, wie weiter unten noch näher erläutert werden wird.

Sie weist auf ihrer dem Femur zuzuwendenden Fläche eine offenmaschige dreidimensionale Raumnetzstruktur auf, durch welche Knochentrapekel ausgehend von der Kortikalis des Femurs hindurchwachsen, um eine Lagefixierung der Zugplatte 3, aber auch der mit ihr nach der Implantation verbundenen Hülse 1 zu erreichen.

Wie ersichtlich, weist die Zugplatte 3 femurwärts ein Widerlager in Form eines Zapfens 18 auf, der hier im selben Winkel geneigt ist wie die Hauptachse der Hülse 1 zur Zugplatte 3.

Hierdurch wird eine besondere Stabilität hinsichtlich etwaig angreifender Biegemomente erzielt.

Fig. 3 zeigt eine Ansicht der Zugschraube 4. Diese weist ausgehend von ihrem Kopf 19 vier Abschnitte auf. Zunächst schließt sich dem Kopf 19 ein zylindrischer Abschnitt 15 mit verringertem Durchmesser gegenüber dem sich daran anschließenden zylindrischen Abschnitt 22 auf. Dieser wiederum folgt ein Gewindeabschnitt 7', dem sich ein weiterer Gewindeabschnitt 14' anschließt. Der Gewindeabschnitt 7' arbeitet mit dem Innengewinde 7 in der Hülse 1 zusammen, wenn nämlich die Zugschraube 4 durch die Zugplatte 3 und die Bohrung in der Kortikalis des Femurs durch die Durchbohrung 5 in der Hülse 1 hindurchgeführt wird. Die Zugschraube 4 zieht sozusagen die Hülse 1 vermittels der Zugplatte 3 nach lateral in ihre Implantationslage. Der durchmesserreduzierte Abschnitt 15 dient zur Feineinstellung der Lage der Zugschraube 4 in der Zugplatte 3 in deren Durchbohrung 21.

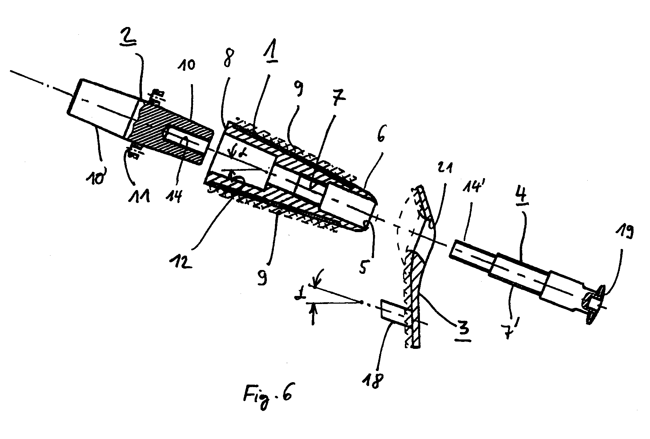

Der distale Gewindeabschnitt 14' der Zugschraube 4 arbeitet mit dem Adapter 2 zusammen, wie er beispielhaft in Fig. 5 teilweise im Schnitt dargestellt ist. Der Adapter 2 besteht vorliegend aus zwei Steckkonen 10 und 10' die voneinander wegragen. Der Steckkonus 10 ist dazu vorgesehen, in die Steckhülse 12 der Hülse gesetzt zu werden und dort unter der Wirkung der entstehenden konischen Klemmverbindung zu verharren. Zur weiteren Lagesicherung ist im Steckkonus 10 eine Sackbohrung 13 mit einem Innengewinde 14 vorgesehen. Mit diesem Innengewinde 14 wird der Gewindeabschnitt 14' der Zugschraube 4 verschraubt.

Mit dem anderen Steckkonus 10' ist in herkömmlicherweise eine künstliche Gelenkkugel verbindbar, ebenfalls unter der Wirkung einer zwischen dem Konus 10' und einer Steckhülse in der Gelenkkugel herzustellenden konischen Klemmverbindung.

Im Bereich der Basen der Konen 10 und 10' des Adapters 2 ist ein umlaufender Flansch 11 ausgebildet. Dieser Flansch dient - wie weiter oben bereits ausgeführt - nicht dazu, eine Ablastung auf der Resektionsfläche des Femurs zu bilden. Dieser Flansch dient lediglich der Rotationssicherung. Zu diesem Zweck sind in dem Flansch 11 einige periphere Bohrungen 23 vorgesehen, durch welche hindurch geeignete Befestigungsmittel (nicht dargestellt) gesetzt werden können, um eine Rotationssicherung durchzuführen. Beispielsweise könnten kleine Schrauben in den Femur durch die Bohrungen 23 hindurchgeführt werden. Alternativ hierzu oder zusätzlich zu dieser Befestigungsmöglichkeit ist vorgesehen, daß die nach außen weisenden Flächen des Flansches 11 mit einer offenmaschigen dreidimensionalen Raumnetzstruktur 16 belegt sind. Nach der Implantation der Endoprothese werden Knochentrapekel von der Resektionsfläche des Femurs in diese Raumstrukturen hineinwachsen bzw. diese durchwachsen und so zu einer Rotationsstabilität führen.

Bevorzugt erfolgt die Implantation der Endoprothese so, daß zwischen der Resektionsfläche und dem Flansch 11 ein Spalt von beispielsweise 1 mm frei bleibt. Dieser Spalt kann problemlos von den Knochentrapekeln überwundet werden, andererseits jedoch bei einem Revisionseingriff leicht durch ein in diesen Spalt hineinzuführendes Sägeblatt wieder aufgetrennt werden.

Aus Fig. 6 ergibt sich schematisch der Effekt der Zusammenwirkung der vorbeschriebenen Komponenten der Endoprothese.

Bei der Implantation befindet sich zwischen der Zugplatte 3 und der Hülse 1 die laterale Kortikalis des Femurs (nicht dargestellt). Die Zugschraube 4 wird durch die Durchbohrung 21 in der Zugplatte 3 und durch die Bohrung in der Kortikalis in die Hülse 1 geführt. Sodann wird der Gewindeabschnitt 7' der Zugschraube 4 mit dem Innengewinde 7 in der Hülse 1 verschraubt, wobei die Hülse 1 dann nach außen (lateral) gezogen wird. Danach kann der Adapter 2 an die Hülse 1 herangebracht werden unter Einsetzen des Steckkonus 10 des Adapters 2 in die Steckhülse 12 in der Hülse 1. Dies geschieht im dargestellten Fall durch ein Einschrauben des Adapters, wobei der Gewindeabschnitt 14' der Zugschraube 4 mit dem Innengewinde 14 in Steckkonus 10 verschraubt wird.

Eine Rotationsstabilität der Hülse 1 wird von vorn herein durch die ovale Querschnittsform erzielt, welche sich vorliegend aus der Belegung der Außenflächen der Hülse 1 mit offenmaschigen dreidimensionalen Raumnetzstrukturen mit unterschiedlichen Maschenweiten - wie beschrieben - ergibt.

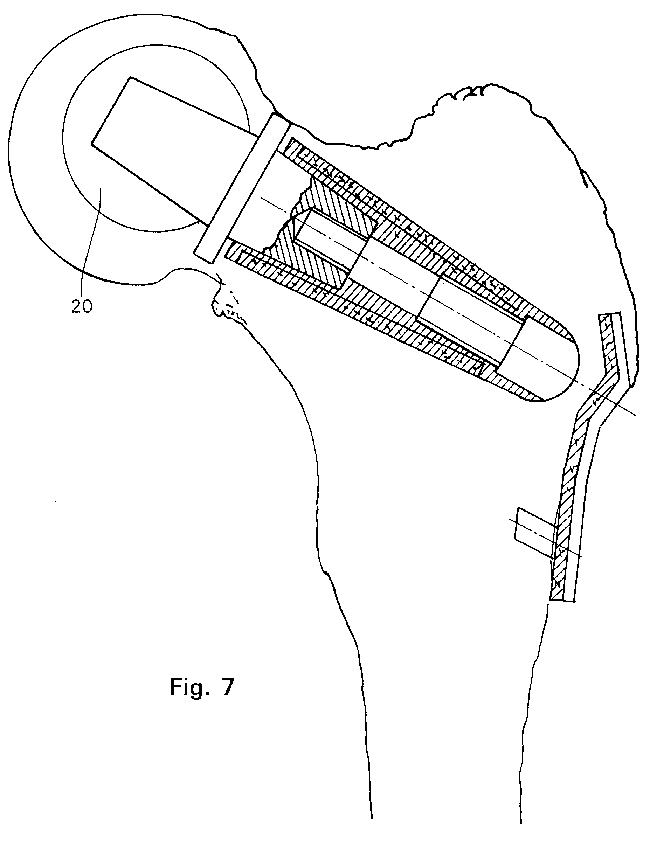

Schließlich sei auf Fig. 7 kurz hingewiesen, aus der heraus sich die Lage der Endoprothese in situ schematisch ergibt. Zusätzlich dargestellt ist eine künstliche Gelenkkugel 20, die mit einer künstlichen Gelenkpfanne zusammenwirkt, um ein künstliches Hüftgelenk zu ergeben. Die Darstellung in Fig. 7 dient lediglich der Anschauung.

## Patentansprüche

1. Schenkelhalsendoprothese für ein künstliches Hüftgelenk, aufweisend
- eine zementlos im oberen Bereich eines Femurs unterhalb des Trochanter majors implantierbare Hülse (1), mit deren proximalen Ende (8) ein Adapter (2) zur Aufnahme einer künstlichen Gelenkkugel (20) verbunden ist, wobei die Hülsenaußenseite zumindest teilweise mit einer offenmaschigen dreidimensionalen Raumnetzstruktur (9) belegt ist, sowie
- eine Zugplatte (3), an der eine Zugschraube (4) angeschlagen ist, die durch eine Durchbohrung (5) im distalen Ende (6) der Hülse (1) in deren Inneres gesetzt und mit einem dort vorgesehenen Gewinde (7) verschraubt ist, wobei
- sie proximalseitig keine Druckplatte zum Einleiten von Druckkräften auf die Kortikalis des Femurs aufweist.

2. Schenkelhalsendoprothese nach Anspruch 1, bei der die Hülse (1) von ihrem proximalen Ende (8) zu ihrem distalen Ende (6) konisch sich verjüngend ausgebildet ist.

3. Schenkelhalsprothese nach Anspruch 1, bei der die Raumnetzstruktur (9) auf den nach kaudal und nach kranial weisenden Hülsenaußenseiten grobmaschig mit Maschenweiten zwischen 3 bis 6 mm ausgebildet ist.

4. Schenkelhalsendoprothese nach einem der Ansprüche 1 bis 3, bei der die Raumnetzstruktur (9) in Richtung ventral und dorsal auf der Hülsenaußenseite feinmaschig mit Maschenweiten zwischen 1 bis 2,5 mm ausgebildet ist.

5. Schenkelhalsendoprothese nach einem der Ansprüche 1 bis 4, bei der der Adapter (2) für die Gelenkkugel im wesentlichen als Doppelsteckkonus (10, 10') ausgebildet ist mit einem an den beiden Konusbasen umlaufenden Flansch (11), wobei im proximalen Bereich der Hülse (1) eine dem einen Steckkonus (10) entsprechend ausgebildete konische Steckhülse (12) vorgesehen ist.

6. Schenkelhalsendoprothese nach Anspruch 5, bei der im der Hülse (1) zuzuwendenden Steckkonus (10) des Adapters (2) eine Sackbohrung (13) mit Innengewinde (14) vorgesehen ist, mit welchem das Ende der mit einem entsprechenden Gewindeabschnitt (14') versehenen Zugschraube (4) zur Arretierung verschraubbar ist.

7. Schenkelhalsprothese nach Anspruch 6, bei der der Gewindeabschnitt (14') am Ende der Zugschraube (4) eine andere Steigung aufweist als das Gewinde (7) im Inneren der Hülse (1).

8. Schenkelhalsprothese nach einem der Ansprüche 1 bis 7, bei der die nach außen weisenden Flächen des Flansches (11) des Adapters (2) mit einer offenmaschigen dreidimensionalen Raumnetzstruktur (16) belegt sind.

9. Schenkelhalsprothese nach einem der Ansprüche 1 bis 8, bei der die dem Femur zuzuwendenden Flächen der Zugplatte (3) mit einer offenmaschigen dreidimensionalen Raumnetzstruktur (17) belegt sind.

10. Shenkelhalsendoprothese nach einem der Ansprüche 1 bis 9, bei der die Zugplatte (3) mit wenigstens einem in Richtung des Femurs weisenden Widerlager in Form eines Zapfens (18) versehen ist, welcher mit der Zugplatte im wesentlichen denselben Winkel α einschließt wie die Hauptachse der Hülse mit der Zugplatte.

## Claims

1. Femoral neck endoprosthesis for an artificial hip joint having
- a shell (1) which can be implanted without cement in the upper region of a femur below the greater trochanter, the proximal end (8) of the shell being connected with an adapter (2) for accommodating an artificial spherical part of a joint (20), whereby the exterior of the shell is at least partially covered with an open-meshed three-dimensional spatial lattice structure (9), as well as
- a draw plate (3) on which a draw-in screw (4) is fastened, which is placed in the interior of the shell (1) through a through bore (5) in its distal end (6), and which is screwed in with a thread (7) provided therein, wherein
- it does not have a fastening plate on the proximal side for introducing compressive forces on the cortex of the femur.

2. Femoral neck endoprosthesis according to claim 1, wherein the shell (1) is constructed as tapering conically from its proximal end (8) to its distal end (6).

3. Femoral neck prosthesis according to claim 1, wherein the spatial lattice structure (9) is constructed with a coarse mesh having mesh widths between 3 to 6 mm on the shell exterior sides which point in caudal and cranial directions.

4. Femoral neck endoprosthesis according to one of claims 1 to 3, wherein the spatial lattice structure (9) is constructed with a fine mesh having mesh widths between 1 to 2.5 mm in ventral and dorsal directions on the shell exterior side.

5. Femoral neck endoprosthesis according to one of claims 1 to 4, wherein the adapter (2) for the spherical joint part is constructed essentially as a double plug cone (10, 10') having an annular flange (11) on the bases of the two cones, wherein a conical plug socket (12) constructed corresponding to the one plug cone (10) is provided in the proximal area of the shell (1).

6. Femoral neck endoprosthesis according to claim 5, wherein a blind bore (13) having an internal thread (14) is provided in the plug core (10) of the adapter (2) facing the shell (1), with which the end of the draw-in screw (4), provided with a corresponding thread section (14'), can be screwed in for arrestation.

7. Femoral neck prosthesis according to claim 6, wherein the thread section (14') at the end of the draw-in screw (4) has a different pitch than the thread (7) in the interior of the shell (1).

8. Femoral neck prosthesis according to one of claims 1 to 7, wherein outward facing surfaces of the flange (11) of the adapter (2) are covered with an open-meshed three-dimensional spatial lattice structure (16).

9. Femoral neck prosthesis according to one of claims 1 to 8, wherein surfaces of the draw plate (3) facing the femur are covered with an open-meshed three-dimensional spatial lattice structure (17).

10. Femoral neck endoprosthesis according to one of claims 1 to 9, wherein the draw plate (3) is provided with at least one abutment in a form of a peg (18) pointing in the direction of the femur, which essentially forms the same angle α with the draw plate as the main axis of the shell with the draw plate.

## Revendications

1. Endoprothèse de col du fémur pour une articulation de hanche artificielle, comprenant : présentant :
- un manchon (1) implantable sans ciment dans la partie supérieure du fémur en dessous du petit trochanter et à l'extrémité proximale (8) duquel est relié un adaptateur (2) destiné à recevoir une tête d'articulation artificielle (20), l'extérieur du manchon étant recouvert au moins en partie d'une structure réticulaire à mailles ouvertes en trois dimensions, ainsi que
- une plaque de tension (3) sur laquelle est fixée une vis de tension (4) introduite à l'intérieur du manchon (1) par un perçage (5) à l'extrémité distale (6) de ce dernier et vissée au moyen d'un filetage (7) prévu par le manchon,
- la prothèse ne présentant du côté proximal aucune plaque d'appui servant à appliquer des forces de compression dans la corticale du fémur.

2. Endoprothèse de col du fémur selon la revendication 1, dans laquelle le manchon (1) est effilé sous forme conique de son extrémité proximale (8) à son extrémité distale (6).

3. Endoprothèse de col du fémur selon la revendication 1, dans laquelle la structure réticulaire en trois dimensions (9) est formée sur les surfaces extérieures du manchon dirigées dans les sens caudal et crânial avec une ouverture de mailles grossière d'une taille de 3 à 6 mm.

4. Endoprothèse de col du fémur selon l'une des revendications 1 à 3, dans laquelle la structure réticulaire en trois dimensions (9) est formée sur les surfaces extérieures du manchon dirigées dans les sens ventral et dorsal avec une ouverture de mailles fine d'une taille de 1 à 2,5 mm.

5. Endoprothèse de col du fémur selon l'une des revendications 1 à 4, dans laquelle d'adaptateur (2) pour tête d'articulation se présente essentiellement sous la forme d'une double broche conique (10, 10') avec une bride (11) entourant la base des deux cônes, une douille d'emmanchement conique (12) correspondant à l'une des broches coniques (10) étant prévue dans la zone proximale du manchon (1).

6. Endoprothèse de col du fémur selon la revendication 5, dans laquelle il est prévu dans la broche conique (10) de l'adaptateur (2) destinée à être dirigée vers le manchon (1) un trou borgne (13) avec un taraudage (14) dans lequel l'extrémité d'une vis de tension (4) munie d'une section filetée (14') correspondante peut se visser pour réaliser un blocage.

7. Endoprothèse de col du fémur selon la revendication 6, dans laquelle le pas de la section filetée (14') à l'extrémité de la vis de tension (4) est différent de celui du taraudage (7) à l'intérieur du manchon (1).

8. Endoprothèse de col du fémur selon l'une des revendications 1 à 7, dans lequel les surfaces de la bride (11) de l'adaptateur (2) dirigées vers l'extérieur sont revêtues d'une structure réticulaire en trois dimensions (16) à mailles ouvertes.

9. Endoprothèse de col du fémur selon l'une des revendications 1 à 8, dans laquelle les surfaces de la plaque de tension (3) destinées à être dirigées vers le fémur sont revêtues d'une structure réticulaire en trois dimensions (17) à mailles ouvertes.

10. Endoprothèse de col du fémur selon l'une des revendications 1 à 9, dans laquelle la plaque de tension (3) est munie d'au moins une butée sous la forme d'un pion (18) pointant en direction du fémur qui fait avec la plaque de tension un angle α sensiblement égal à celui que fait l'axe principal du manchon avec la plaque de tension.
